# EUROPEAN PATENT APPLICATION

(11) **EP 4 170 015 A1**
(43) Date of publication of application: **26.04.2023**
(21) Application number: 21913710.6
(22) Date of filing: 30.11.2021
(51) Int. Cl.: C12N 1/20, C12N 1/38, C12P 7/18, C12R 1/19

(54) **METHOD FOR PREPARING XYLITOL BY FERMENTING XYLOSE SECONDARY MOTHER LIQUOR**

(30) Priority: 28.12.2020 CN 202011606471
(71) Applicant: Zhejiang Huakang Pharmaceutical Co., Ltd., Quzhou, Zhejiang 324302 (CN); Zhejiang University of Technology, Hangzhou, Zhejiang 310014 (CN)
(72) Inventor: ZENG, Xuhao, Quzhou City, Zhejiang 324302 (CN); LUO, Jiaxing, Quzhou City, Zhejiang 324302 (CN); WANG, Jing, Quzhou City, Zhejiang 324302 (CN); HU, Changhui, Quzhou City, Zhejiang 324302 (CN); FANG, Shuncheng, Quzhou City, Zhejiang 324302 (CN); LIU, Zhiqian, Quzhou City, Zhejiang 324302 (CN); CAI, Xue, Quzhou City, Zhejiang 324302 (CN); ZHANG, Xiaojian, Quzhou City, Zhejiang 324302 (CN); ZHENG, Yuguo, Quzhou City, Zhejiang 324302 (CN); LI, Mian, Santa Clara, California 95054 (US)
(74) Representative: Sun, Yiming
(86) International application number: PCT/CN2021/134586
(87) International publication number: WO 2022/142969

(57) **Abstract**

The present invention relates to a method of preparing xylitol by using a xylose secondary mother liquor through fermentation. In the method, fermentation treatment is performed on a mixed liquid of the xylose secondary mother liquor and a xylose concentrate, and the xylose secondary mother liquor is subsequently batch-fed instead of glucose in the fermentation process. Thus, a glucose content in the fermentation system is stabilized, and some xylose is provided at the same time. In this way, the use amount of glucose and costs are reduced, and the system xylose can be relatively stabilized, thus improving the entire conversion rate. In the present invention, adding the xylose secondary mother liquor by mixed fermentation and batch feeding and the like enables the contents of heterosaccharides such as arabinose and mannose in the fermentation system to be always within a range of metabolization by fermenting strains, facilitating subsequent purification and improving the xylitol purity.

## Description

### TECHNICAL FIELD

The present invention relates to the field of sugar alcohol preparation technologies, and in particular to a method of preparing xylitol by using a xylose secondary mother liquor through fermentation.

### BACKGROUND

In a xylose preparation process, raw materials such as corn cob go through processes such as acid hydrolysis, decolorization, ion exchange, concentration, crystallization, centrifugation and drying to produce a crystal xylose. During a centrifugation process, a large quantity of xylose mother liquor will be obtained. The mother liquor is treated by chromatographic separation to extract xylose again, while a raffinate, i.e. a xylose secondary mother liquor, is obtained in the chromatographic separation. The xylose secondary mother liquor contains many monosaccharides, such as glucose, arabinose, galactose, mannose, and xylose and the like. Due to different production conditions and different batches, the contents will change. Hence, it is very difficult to achieve reasonable utilization on it. At present, the xylose secondary mother liquor is mostly used as a mixed syrup for production of a caramel pigment, resulting in low added value.

In a current method of preparing xylitol by using biological fermentation, fermentation is performed by using high-efficiency saccharomycetes or genetically engineered bacteria of escherichia coli with a hemicellulose hydrolysate or glucose as a fermentation raw material, as shown in the patents with the publication numbers CN105671013A, CN110835621A and CN106661540A. In the existing fermentation technologies, in order to satisfy fermentation production efficiency, an additional material is usually fed in this process and mostly, glucose is added to provide a carbon source for strain production and fermentation, which increases the fermentation costs and the difficulty of fermentation process control. Because there is usually glucose residue at the endpoint of the fermentation, a content of a reducing sugar in a fermentation liquid is high, which is unfavorable for subsequent fine treatment of the fermentation liquid.

In the patent with the publication number CN101921810A, a candida for which L-arabinose cannot be used as a carbon source is used to perform fermentation on a xylose mother liquor and consume glucose and galactose in the mother liquor so as to obtain a mixed crystal of xylitol and L-arabinose by using processes such as decolorization, ion exchange, concentration and crystallization. In the patent with the publication number CN101705253A, an alcohol yeast and an xylitol yeast are used to perform fermentation on a xylose mother liquor in sequence to obtain ethanol, xylitol and arabinose respectively. In the patent with the publication number CN101857523A, a xylose mother liquor is used to produce xylitol and arabitol at the same time. In these researches, a xylose mother liquor is used to prepare xylitol by fermentation. Compared with the xylose mother liquor, the xylose secondary mother liquor has a lower xylose content which is about 10% (about 50%-60% in the xylose mother liquor), and thus, it is not applicable to direct conversion of xylitol. Further, because the xylose secondary mother liquor has a higher mother liquor solid content, it is to be diluted for fermentation, which further reduces the xylose concentration. Thus, the fermentation liquid has an excessively low xylitol concentration and has no value for extraction and purification.

The above researches have the following common problem: with the xylose mother liquor simply used as a carbon source and a fermentation raw material, the fermentation efficiency is low, and the fermentation utilization of the xylose secondary mother liquor is not applicable. For the purpose of preparing xylitol using a xylose secondary mother liquor, the existing technology is to be further improved.

### SUMMARY

In order to solve the above technical problems, the present invention provides a method of preparing xylitol by using a xylose secondary mother liquor through fermentation, in which the xylose secondary mother liquor and a xylose concentrate are mixed at a ratio for fermentation while an initial glucose content of the material is increased to reduce an amount of subsequent glucose batch feed. The xylose secondary mother liquor is subsequently batch-fed instead of glucose to solve the following problems: the xylose content in the xylose secondary mother liquor is low, the xylitol conversion rate is low, the fermentation operation steps are complex and production costs are high. Hence, the added value of the xylose secondary mother liquor is increased.

The present invention is implemented by providing a method of preparing xylitol by using a xylose secondary mother liquor through fermentation, which includes the following steps:
at step 1, xylose concentrate preparation: adding a base liquid to a pre-ion exchange liquid of a xylose hydrolysate, then performing decolorization using an activated carbon to obtain a fine xylose liquid by filtration, and then performing low-temperature vacuum concentration until a concentrate refractive index is about 60% to 65% so as to obtain a xylose concentrate;
at step 2, strain activation and seed liquid preparation: selecting recombinant escherichia coli strains IS5-M, activating the strains by using an LB plate and an LB liquid medium to inoculate into a seed medium and cultivate to OD₆₀₀=5 to 6 so as to obtain a seed liquid;
at step 3, fermentation tank propagation: adding a sterilized fermentation medium to a fermentation tank and adding the seed liquid to cultivate to OD₆₀₀ > 20;
at step 4, first batch feed fermentation: adding a mixed liquid obtained by mixing the xylose concentrate and the xylose secondary mother liquor at a ratio to the fermentation tank to perform feed fermentation, such that a xylose concentration in a fermentation system is maintained at 85g/L to 90g/L; when a glucose concentration in the system is less than 10g/L, batch-feeding the xylose secondary mother liquor to control the glucose concentration to 10g/L, where a total glucose addition amount is 35% to 40% of a xylose mass; during the fermentation process, batch-feeding 0.2L to 0.5L of a high concentration solution prepared by using 120g to 130g of a corn steep liquor dry powder liquid at the same time;
at step 5, second batch feed fermentation: when the xylose concentration in the first batch fermentation is < 30g/L, performing the second batch feed fermentation using the method in step 4 until a xylitol concentration in the fermentation liquid has no change.

Furthermore, the step 1 specifically includes: adding Ca(OH)₂ of 0.4% to 0.5% to the pre-ion exchange liquid of the xylose hydrolysate, and stirring at a speed of 30rpm to 50rpm for 15min to 30min to control pH to 3.0 to 3.5, and then adding the activated carbon of a mass ratio of 0.3% to 0.5% for performing decolorization with a control temperature of 60°C to 70°C and stirring at a speed of 30rpm to 60rpm for 60min to 90min. Furthermore, in step 1, the conditions of the low-temperature vacuum concentration are that a vacuum pump pressure is -0.085MPa to -0.095MPa, a water bath temperature is 60°C to 70 °C, a rotation speed is 50rpm to 60rpm, and a condensate flow rate is 150mL/min to 200mL/min.

Furthermore, in step 2, the strains are recombinant escherichia coli IS5-M. The strains are recorded in the patent with the publication number CN105671013A.

Furthermore, in step 2, ingredients of the LB liquid medium are peptone 10g/L, yeast powder 5g/L, sodium chloride 10g/L and solid agar 1.5% to 2%; ingredients of the seed medium are: peptone 7.5g/L, yeast powder 7.5g/L, sodium chloride 10g/L, and glucose 20g/L.

Furthermore, in step 3, ingredients of the fermentation medium are: glucose 10g/L, corn steep liquor dry powder 24g/L, sodium chloride 0.5g/L, KH2PO4 3g/L, Na2HPO4•12H2O 9g/L and NH4Cl 1g/L; cultivation conditions of the fermentation tank are: 37°C, 400rpm, pH6.5-7 after ammonia water adjustment, dissolved oxygen 30% to 35%.

Furthermore, in step 3, the cultivation lasts for 9.5h to 11h.

Furthermore, in step 4, a mixing ratio of the xylose concentrate to the xylose secondary mother liquor is 7to 9:1to 3.

Furthermore, in step 4, fermentation conditions of the first batch feed fermentation are: 30°C, pH7, dissolved oxygen 20% to 25%.

Furthermore, the entire fermentation time is about 75h to 85h.

Compared with the prior arts, the method of preparing xylitol using a xylose secondary mother liquor through fermentation according to the present invention has the following features:
(1) Mixing the xylose secondary mother liquor and the xylose concentrate at a ratio can increase a content of an available carbon source at the early period of the fermentation, thus helping growth and propagation of the strains at the early period, and improving the xylitol conversion rate.
(2) The xylose secondary mother liquor is batch-fed instead of glucose to maintain a stable glucose content of the fermentation system while providing some xylose. In this way, a use amount of glucose can be reduced and the costs are also lowered. Further, relative stability of xylose in the system can be maintained and the entire conversion rate can be improved.
(3) There are smaller contents of inhibitors in the xylose secondary mother liquor. The xylose secondary mother liquor does not affect strain development and can be used without sterilization.
(4) Two batches of feed fermentations can maintain relative stability of the xylose content in the fermentation system, helping strain conversion and increasing the xylitol concentration in the fermentation liquid. Further, a lower conversion rate resulting from growth inhibition of strains under high osmotic conditions due to excessively high concentration of saccharides such as xylose at the early period of the fermentation is avoided.
(5) Adding the xylose secondary mother liquor by mixed fermentation and batch feeding and the like enables the contents of heterosaccharides such as arabinose and mannose in the fermentation system to be always within a range of metabolization by fermenting strains, facilitating subsequent purification and improving the xylitol purity.

### DETAILED DESCRIPTION OF THE EMBODIMENTS

In order to make the technical problems, the technical solutions and the beneficial effects of the present invention clearer, the present invention will be further described in details in combination with the specific embodiments. It should be understood that the specific embodiments described herein are used only to interpret the present invention rather than limit the present invention.

A preferred embodiment of a method of preparing xylitol by using a xylose secondary mother liquor through fermentation according to the present invention includes the following steps.

At step 1, xylose concentrate preparation: a base liquid is added to a pre-ion exchange liquid of a xylose hydrolysate, then decolorization is performed using an activated carbon so as to obtain a fine xylose liquid by filtration, and then low-temperature vacuum concentration is performed until a concentrate refractive index is about 60% to 65% so as to obtain a xylose concentrate.

At step 2, strain activation and seed liquid preparation: recombinant escherichia coli strains IS5-M are selected, and the strains are activated by using an LB plate and an LB liquid medium and inoculated into a seed medium and cultivated to OD₆₀₀=5 to 6 so as to obtain a seed liquid;

At step 3, fermentation tank propagation: a sterilized fermentation medium is added to a fermentation tank and the seed liquid obtained in step 2 is added and cultivated to OD₆₀₀ > 20.

At step 4, first batch feed fermentation: a mixed liquid obtained by mixing the xylose concentrate prepared in step 1 and the xylose secondary mother liquor at a ratio is added to the fermentation tank to perform feed fermentation, such that a xylose concentration in a fermentation system is maintained at 85g/L to 90g/L; when a glucose concentration in the system is less than 10g/L, the xylose secondary mother liquor is batch-fed to control the glucose concentration to 10g/L, where a total glucose addition amount is 35% to 40% of a xylose mass; during the fermentation process, 0.2L to 0.5L of a high concentration solution prepared by using 120g to 130g of a corn steep liquor dry powder liquid is batch-fed at the same time.

At step 5, second batch feed fermentation: when the xylose concentration in the first batch fermentation is < 30g/L, the second batch feed fermentation is performed using the method in step 4 until a xylitol concentration in the fermentation liquid has no change.

Ingredient contents of the xylose mother liquor and the xylose secondary mother liquor are detected with results shown in Table 1.

**Table 1 Comparison of ingredient contents of the xylose mother liquor and the xylose secondary mother liquor**

| | Refrac tive index (%) | Xylose conce ntratio n (g/L) | Xylose ratio (%) | Glucos e conce ntratio n (g/L) | Glucos e ratio (%) | Acetic acid conce ntratio n (g/L) | Furfur al conce ntratio n (g/L) | HMF conce ntratio n (g/L) |
|---|---|---|---|---|---|---|---|---|
| Xylose mother liquor | 73 | 407.67 | 57.90 | 74.37 | 12.89 | 0.34 | 2.83 | 18.95 |
| Xylose secondary mother liquor | 69 | 75.95 | 11.17 | 142.93 | 33.94 | 0.83 | 2.70 | 0 |

It can be known from the above Table that in the xylose mother liquor, the xylose concentration is higher and the contents of other saccharides are smaller; in the xylose secondary mother liquor, the xylose concentration is lower but the glucose content is higher, and there are smaller contents of inhibitors such as acetic acid, furfural and HMF and the like. Compared with the xylose mother liquor, the xylose secondary mother liquor is suitable for xylitol fermentation and thus is a good fermentation raw material replacing glucose.

A series of raw material liquids for fermentation can be obtained by mixing the xylose concentrate and the xylose secondary mother liquor at a ratio. By detecting the mixed raw material liquids, the contents of the sugar alcohol ingredients of the mixed raw material liquids can be obtained, with results shown in Table 2.

**Table 2**

| Ratio (xylose concentrate:xylose mother liquor) | Xylose concentration (g/L) | Glucose concentration (g/L) | Arabinose concentration (g/L) | Mann ose conce ntrati on (g/L ) |
|---|---|---|---|---|
| 9:1 | 503 | 46 | 57 | 4 |
| 8:2 | 455 | 57 | 68 | 8 |
| 7:3 | 408 | 67 | 80 | 11 |

Other ratios may also be used for the fermentation of the present system. Because the xylose concentration is lower and the space required for fermentation is larger with lower economic benefits, redundant descriptions are not made herein. But these ratios still fall within the scope of protection of the present invention.

The xylose concentrate used in the present invention may be a corn cob hydrolysate, or another biomass raw material rich in hemicelluloses, such as sugarcane bagasse and paper-making wastes and the like. The xylose secondary mother liquor in the present invention may be replaced with another sugar paste liquid rich in glucose.

The method of the present invention will be further described below in combination with specific embodiments.

### Embodiment 1

The first embodiment of the method of preparing xylitol by using a xylose secondary mother liquor through fermentation according to the present invention includes the following steps.

At step 11, xylose concentrate preparation: Ca(OH)₂ of 0.4% was added to a pre-ion exchange liquid of a xylose hydrolysate, stirred at a speed of 50rpm for 15min and controlled to pH3.2, and then an activated carbon with a mass ratio of 0.4% was added for performing decolorization with a control temperature of 60°C and stirring was performed at a speed of 60rpm for 90min. After decolorization was completed, a fine xylose liquid was obtained by filtration, and then low-temperature vacuum concentration was performed with a vacuum pump pressure of -0.095MPa, a water bath temperature of 60°C, a rotation speed of 60rpm and a condensate flow rate of 200mL/min until a concentrate refractive index was about 65%, so as to finally obtain a xylose concentrate applicable to fermentation.

At step 12, strain activation and seed liquid preparation: recombinant escherichia coli strains IS5-M were selected, and the strains were activated by using an LB plate and an LB liquid medium and inoculated into a seed medium and cultivated to obtain a seed liquid of OD₆₀₀=5.

At step 13, fermentation tank propagation: 3.5L of sterilized fermentation medium was added to a 15L fermentation tank and 0.5L of seed liquid prepared in step 12 was added to perform cultivation to OD₆₀₀=21 under the following conditions: 37°C, 400rpm, pH6.5 to 7 after ammonia water adjustment, dissolved oxygen 30% to 35%, working volume 4.5L, cultivation time about 10h.

At step 14, first batch feed fermentation: 1L of mixed liquid obtained by mixing the xylose concentrate prepared at step 11 and the xylose secondary mother liquor at a ratio of 9:1 was added to the fermentation tank to perform feed fermentation, such that a xylose concentration in a fermentation system was maintained at 90g/L. The fermentation conditions were: 30°C, pH7 and dissolved oxygen 20% to 25%. As theoretically calculated, 200g of glucose was to be added to provide sufficient coenzyme NADPH. Since there was some glucose in the mixed liquid, the xylose secondary mother liquor was needed to replenish remaining glucose by batch feed, totaling 1.1L of xylose secondary mother liquor to be replenished. The xylose secondary mother liquor was replenished by batch feed and the glucose content was controlled to about 10g/L. During a fermentation process, about 0.4L of a high concentration solution prepared by using 120g to 130g of a corn steep liquor dry powder liquid was batch-fed at the same time. The working volume was about 7L after fermentation was completed.

At step 15, second batch feed fermentation: when the xylose concentration in the first batch fermentation was < 30g/L, the second batch feed fermentation was performed using the method in step 14 to add 1.5L of mixed liquid prepared using the xylose concentrate and the xylose secondary mother liquor (mixing the xylose concentrate and the xylose secondary mother liquor at a ratio of 9:1) to the fermentation tank, and at this time, the xylose concentration in the system was about 88g/L. As theoretically calculated, 300g of glucose was to be added to provide sufficient coenzyme NADPH. The xylose secondary mother liquor was provided instead of glucose such that 1.6L of xylose secondary mother liquor was replenished by batch feed. The xylose secondary mother liquor was replenished by batch feed and the glucose content was controlled to about 10g/L. During a fermentation process, about 0.4L of a high concentration solution prepared by using 120g to 130g of a corn steep liquor dry powder liquid was batch-fed at the same time. The fermentation ended when the xylitol concentration had no change. The working volume was about 10.5L after fermentation was completed.

To the fermentation system, a total of 1465g of xylose was added, saving a total of 500g of glucose.

HPLC detection was performed on the fermentation liquid after the fermentation of the present embodiment to obtain a xylitol concentration of 121.4g/L, a conversion rate of 87% and arabinose residues of 5.5g/L in the fermentation liquid, and no heterosaccharides such as glucose and mannose were detected.

### Embodiment 2

The second embodiment of the method of preparing xylitol by using a xylose secondary mother liquor through fermentation according to the present invention includes the following steps.

At step 21, xylose concentrate preparation: Ca(OH)₂ of 0.5% was added to a pre-ion exchange liquid of a xylose hydrolysate, stirred at a speed of 30rpm for 30min and controlled to pH3.0, and then an activated carbon with a mass ratio of 0.5% was added for performing decolorization with a control temperature of 70°C and stirring was performed at a speed of 30rpm for 60min. After decolorization was completed, a fine xylose liquid was obtained by filtration, and then low-temperature vacuum concentration was performed with a vacuum pump pressure of -0.085MPa, a water bath temperature of 70°C, a rotation speed of 50rpm and a condensate flow rate of 150mL/min until a concentrate refractive index was about 60%, so as to finally obtain a xylose concentrate applicable to fermentation.

At step 22, strain activation and seed liquid preparation: recombinant escherichia coli strains IS5-M were selected, and the strains were activated by using an LB plate and an LB liquid medium and inoculated into a seed medium and cultivated to obtain a seed liquid of OD₆₀₀=5.5.

At step 23, fermentation tank propagation: 4.0L of sterilized fermentation medium was added to a 15L fermentation tank and 0.5L of seed liquid prepared in step 22 was added to perform cultivation to OD₆₀₀=23 under the following conditions: 37 °C, 400rpm, pH6.5 to 7 after ammonia water adjustment, dissolved oxygen 30% to 35%, working volume 5.0L, cultivation time about 10h.

At step 24, first batch feed fermentation: 1.4L of mixed liquid obtained by mixing the xylose concentrate prepared at step 21 and the xylose secondary mother liquor at a ratio of 7:3 was added to the fermentation tank to perform feed fermentation, such that a xylose concentration in a fermentation system was maintained at 89g/L. The fermentation conditions were: 30°C, pH7 and dissolved oxygen 20% to 25%. As theoretically calculated, 230g of glucose was to be added to provide sufficient coenzyme NADPH. Since there was some glucose in the mixed liquid, the xylose secondary mother liquor was needed to replenish remaining glucose by batch feed, totaling 0.95L of xylose secondary mother liquor to be replenished. The xylose secondary mother liquor was replenished by batch feed and the glucose content was controlled to about 10g/L. During a fermentation process, about 0.5L of a high concentration solution prepared by using 120g to 130g of a corn steep liquor dry powder liquid was batch-fed at the same time. The working volume was about 8L after fermentation was completed.

At step 25, second batch feed fermentation: when the xylose concentration in the first batch fermentation was < 30g/L, the second batch feed fermentation was performed using the method in step 24 to add 2.2L of mixed liquid prepared using the xylose concentrate and the xylose secondary mother liquor (mixing the xylose concentrate and the xylose secondary mother liquor at a ratio of 7:3) to the fermentation tank, and at this time, the xylose concentration in the system was about 88g/L. As theoretically calculated, 360g of glucose was to be added to provide sufficient coenzyme NADPH. The xylose secondary mother liquor was provided instead of glucose such that 1.5L of xylose secondary mother liquor was replenished by batch feed. The xylose secondary mother liquor was replenished by batch feed and the glucose content was controlled to about 10g/L. During a fermentation process, about 0.5L of a high concentration solution prepared by using 120g to 130g of a corn steep liquor dry powder liquid was batch-fed at the same time. The fermentation ended when the xylitol concentration had no change. The working volume was about 12.5L after fermentation was completed.

To the fermentation system, a total of 1657g of xylose was added, saving a total of 590g of glucose.

HPLC detection was performed on the fermentation liquid after the fermentation of the present embodiment to obtain a xylitol concentration of 114.1g/L, a conversion rate of 86% and arabinose residues of 5.4g/L in the fermentation liquid, and no heterosaccharides such as glucose and mannose were detected.

### Embodiment 3

The third embodiment of the method of preparing xylitol by using a xylose secondary mother liquor through fermentation according to the present invention includes the following steps.

At step 31, xylose concentrate preparation: Ca(OH)₂ of 0.4% was added to a pre-ion exchange liquid of a xylose hydrolysate, stirred at a speed of 50rpm for 15min and controlled to pH3.5, and then an activated carbon with a mass ratio of 0.3% was added for performing decolorization with a control temperature of 60°C and stirring was performed at a speed of 60rpm for 90min. After decolorization was completed, a fine xylose liquid was obtained by filtration, and then low-temperature vacuum concentration was performed with a vacuum pump pressure of -0.095MPa, a water bath temperature of 60°C, a rotation speed of 60rpm and a condensate flow rate of about 200mL/min until a concentrate refractive index was about 65%, so as to finally obtain a xylose concentrate applicable to fermentation.

At step 32, strain activation and seed liquid preparation: recombinant escherichia coli strains IS5-M were selected, and the strains were activated by using an LB plate and an LB liquid medium and inoculated into a seed medium and cultivated to obtain a seed liquid of OD₆₀₀=6.

At step 33, fermentation tank propagation: 3.5L of sterilized fermentation medium was added to a 15L fermentation tank and 0.5L of seed liquid prepared in step 32 was added to perform cultivation to OD₆₀₀=21 under the following conditions: 37°C, 400rpm, pH6.5 to 7 after ammonia water adjustment, dissolved oxygen 30% to 35%, working volume 4.5L, cultivation time about 9.5h.

At step 34, first batch feed fermentation: 1L of mixed liquid obtained by mixing the xylose concentrate prepared at step 31 and the xylose secondary mother liquor at a ratio of 9:1 was added to the fermentation tank to perform feed fermentation, such that a xylose concentration in a fermentation system was maintained at 90g/L. The fermentation conditions were: 30°C, pH7 and dissolved oxygen 20% to 25%. As theoretically calculated, 200g of glucose was to be added to provide sufficient coenzyme NADPH. Since there was some glucose in the mixed liquid, the xylose secondary mother liquor was needed to replenish remaining glucose by batch feed, totaling 1.1L of xylose secondary mother liquor to be replenished. The xylose secondary mother liquor was replenished by batch feed and the glucose content was controlled to about 10g/L. During a fermentation process, about 0.4L of a high concentration solution prepared by using 120g to 130g of a corn steep liquor dry powder liquid was batch-fed at the same time. The working volume was about 7L after fermentation was completed.

At step 35, second batch feed fermentation: when the xylose concentration in the first batch fermentation was < 30g/L, the second batch feed fermentation was performed using the method in step 34 to add 2.0L of mixed liquid prepared using the xylose concentrate and the xylose secondary mother liquor (mixing the xylose concentrate and the xylose secondary mother liquor at a ratio of 7:3) to the fermentation tank, and at this time, the xylose concentration in the system was about 90g/L. As theoretically calculated, 330g of glucose was to be added to provide sufficient coenzyme NADPH. The xylose secondary mother liquor was provided instead of glucose such that 1.4L of xylose secondary mother liquor was replenished by batch feed. The xylose secondary mother liquor was replenished by batch feed and the glucose content was controlled to about 10g/L. During a fermentation process, about 0.5L of a high concentration solution prepared by using 120g to 130g of a corn steep liquor dry powder liquid was batch-fed at the same time. The fermentation ended when the xylitol concentration had no change. The working volume was about 11L after fermentation was completed.

To the fermentation system, a total of 1511g of xylose was added, saving a total of 530g of glucose.

HPLC detection was performed on the fermentation liquid after the fermentation of the present embodiment to obtain a xylitol concentration of 122.4g/L, a conversion rate of 89% and arabinose residues of 5.6g/L in the fermentation liquid, and no heterosaccharides such as glucose and mannose were detected.

### Embodiment 4

The fourth embodiment of the method of preparing xylitol by using a xylose secondary mother liquor through fermentation according to the present invention includes the following steps.

At step 41, xylose concentrate preparation: Ca(OH)₂ of 0.4% was added to a pre-ion exchange liquid of a xylose hydrolysate, stirred at a speed of 50rpm for 15min and controlled to pH3.3, and then an activated carbon with a mass ratio of 0.3% was added for performing decolorization with a control temperature of 60°C and stirring was performed at a speed of 60rpm for 90min. After decolorization was completed, a fine xylose liquid was obtained by filtration, and then low-temperature vacuum concentration was performed with a vacuum pump pressure of -0.095MPa, a water bath temperature of 60°C, a rotation speed of 60rpm and a condensate flow rate of 180mL/min until a concentrate refractive index was about 65%, so as to finally obtain a xylose concentrate applicable to fermentation.

At step 42, strain activation and seed liquid preparation: recombinant escherichia coli strains IS5-M were selected, and the strains were activated by using an LB plate and an LB liquid medium and inoculated into a seed medium and cultivated to obtain a seed liquid of OD₆₀₀=5.

At step 43, fermentation tank propagation: 4.0L of sterilized fermentation medium was added to a 15L fermentation tank and 0.5L of seed liquid prepared in step 42 was added to perform cultivation to OD₆₀₀=24 under the following conditions: 37 °C, 400rpm, pH6.5 to 7 after ammonia water adjustment, dissolved oxygen 30% to 35%, working volume 4.5L, cultivation time about 11h.

At step 24, first batch feed fermentation: 1.1L of mixed liquid obtained by mixing the xylose concentrate prepared at step 31 and the xylose secondary mother liquor at a ratio of 9:1 was added to the fermentation tank to perform feed fermentation, such that a xylose concentration in a fermentation system was maintained at 90g/L. The fermentation conditions were: 30°C, pH7 and dissolved oxygen 20% to 25%. As theoretically calculated, 220g of glucose was to be added to provide sufficient coenzyme NADPH. Since there was some glucose in the mixed liquid, the xylose secondary mother liquor was needed to replenish remaining glucose by batch feed, totaling 1.2L of xylose secondary mother liquor to be replenished. The xylose secondary mother liquor was replenished by batch feed and the glucose content was controlled to about 10g/L. During a fermentation process, 0.2L of a high concentration solution prepared by using 120g to 130g of a corn steep liquor dry powder liquid was batch-fed at the same time. The working volume was about 7.5L after fermentation was completed.

At step 45, second batch feed fermentation: when the xylose concentration in the first batch fermentation was < 30g/L, the second batch feed fermentation was performed using the method in step 44 to add 1.8L of mixed liquid prepared using the xylose concentrate and the xylose secondary mother liquor (mixing the xylose concentrate and the xylose secondary mother liquor at a ratio of 8:2) to the fermentation tank, and at this time, the xylose concentration in the system was about 88g/L. As theoretically calculated, 330g of glucose was to be added to provide sufficient coenzyme NADPH. The xylose secondary mother liquor was provided instead of glucose such that 1.6L of xylose secondary mother liquor was replenished by batch feed. The xylose secondary mother liquor was replenished by batch feed and the glucose content was controlled to about 10g/L. During a fermentation process, 0.4L of a high concentration solution prepared by using 120g to 130g of a corn steep liquor dry powder liquid was batch-fed at the same time. The fermentation ended when the xylitol concentration had no change. The working volume was about 11L after fermentation was completed.

To the fermentation system, a total of 1588g of xylose was added, saving a total of 550g of glucose.

HPLC detection was performed on the fermentation liquid after the fermentation of the present embodiment to obtain a xylitol concentration of 125.6g/L, a conversion rate of 87% and arabinose residues of 5.8g/L in the fermentation liquid, and no heterosaccharides such as glucose and mannose were detected.

The above embodiments show that different ratios of the xylose concentrate and the xylose secondary mother liquor can be applied to the fermentation system and the batch feeding of the xylose secondary mother liquor can further reduce the use amount of glucose.

### Control embodiment 1

The control embodiment includes the following steps.

At step (D11), xylose concentrate preparation: it is same as the preparation method in step 11 of the first embodiment and will not be repeated herein.

At step (D12), strain activation and seed liquid preparation: it is same as the preparation method in step 12 of the first embodiment and will not be repeated herein.

At step (D13), fermentation tank propagation: 5.5L of sterilized fermentation medium was added to a 15L fermentation tank, and 0.6L of seed liquid prepared in step (D12) was added to perform cultivation to OD₆₀₀=21 under the following conditions: 37°C, 400rpm, pH6.5 to 7 after ammonia water adjustment, dissolved oxygen 30% to 35%, working volume 6.5L, cultivation time about 9.5h.

At step (D14), first batch feed fermentation: the xylose concentrate prepared in step (D11) was directly used to perform feed fermentation. 1.2L of xylose concentrate was added to the fermentation tank such that the xylose concentration in the fermentation system was 85g/L. 260g of glucose was to be added to provide sufficient coenzyme NADPH. During a fermentation process, 0.5L of corn steep liquor dry powder liquid was batch-fed at the same time. The working volume was about 8.4L after the fermentation was completed.

At step (D15), second batch feed fermentation: 1.7L of xylose concentrate was added to the fermentation tank by using the method of the step (D14) such that the xylose concentration in the fermentation system was 88g/L. 380g of glucose was to be added to provide sufficient coenzyme NADPH. During a fermentation process, 0.4L of corn steep liquor dry powder liquid was batch-fed at the same time. The working volume was about 10.5L after the fermentation was completed.

To the fermentation system, a total of 1600g of xylose was added, not saving glucose but consuming 640g of glucose.

HPLC detection was performed on the fermentation liquid after the fermentation of the control embodiment to obtain a xylitol concentration of 124.9g/L, a conversion rate of 82% and arabinose residues of 3.8g/L in the fermentation liquid, and no heterosaccharides such as glucose and mannose were detected.

### Control embodiment 2

The control embodiment includes the following steps.

At step (D21), xylose concentrate preparation: it is same as the preparation method in step 21 of the second embodiment and will not be repeated herein.

At step (D22), strain activation and seed liquid preparation: it is same as the preparation method in step 22 of the second embodiment and will not be repeated herein.

At step (D23), fermentation tank propagation: 4.5L of sterilized fermentation medium was added to a 15L fermentation tank, and 0.6L of seed liquid prepared in step (D22) was added to perform cultivation to OD₆₀₀=22 under the following conditions: 37°C, 400rpm, pH6.5 to 7 after ammonia water adjustment, dissolved oxygen 30% to 35%, working volume 5.5L, cultivation time about 10h.

At step (D24), first batch feed fermentation: 1.2L of mixed liquid obtained by mixing the xylose concentrate prepared in step (D21) and the xylose secondary mother liquor at a ratio of 9:1 was added to the fermentation tank such that the xylose concentration in the fermentation system was 90g/L. No glucose or material containing glucose was replenished subsequently. During a fermentation process, 0.5L of corn steep liquor dry powder liquid was batch-fed at the same time. The working volume was about 7.2L. In the system, the content of glucose was only 7.7g/L. At this time, there is no sufficient glucose to be transformed into NADPH. The xylitol conversion rate was low and the xylose concentration cannot be lowered.

At step (D25), second batch feed fermentation: after about 35h (the ending time of the first batch feed fermentation under normal circumstance), second batch feed fermentation was performed by using the method of the step (D24) to add 1.5L of mixed liquid obtained by mixing the xylose concentrate and the xylose secondary mother liquor (the mixing ratio of the xylose concentrate to the xylose secondary mother liquor was 9:1) to the fermentation tank. At this time, the xylose concentration in the fermentation system was 86g/L. During a fermentation process, 0.5L of corn steep liquor dry powder liquid was batch-fed at the same time. The working volume was about 9.5L. In the system, the content of glucose was only 7.2g/L. No glucose or material containing glucose was replenished subsequently. After about 40h of fermentation, the fermentation was ended by hand.

To the fermentation system, a total of 1358g of xylose was added. No replenishment of glucose results in great reduction of the xylitol conversion rate.

HPLC detection was performed on the fermentation liquid after the fermentation of the control embodiment to obtain a xylitol concentration of 58.6g/L, a conversion rate of 41% and arabinose residues of 2.2g/L in the fermentation liquid, and no heterosaccharides such as glucose and mannose were detected.

### Control embodiment 3

The control embodiment includes the following steps.

At step (D31), xylose concentrate preparation: it is same as the preparation method in step 41 of the fourth embodiment and will not be repeated herein.

At step (D32), strain activation and seed liquid preparation: it is same as the preparation method in step 42 of the fourth embodiment and will not be repeated herein.

At step (D33), fermentation tank propagation: 3.5L of sterilized fermentation medium was added to a 15L fermentation tank, and 0.5L of seed liquid prepared in step (D32) was added to perform cultivation to OD₆₀₀=22 under the following conditions: 37°C, 400rpm, pH6.5 to 7 after ammonia water adjustment, dissolved oxygen 30% to 35%, working volume 4.5L, cultivation time about 10.5h.

At step (D34), full feed fermentation: fermentation was performed by using a mixed liquid prepared by mixing the xylose concentrate and the xylose secondary mother liquor at a ratio of 9:1. 2.5L of mixed liquid was directly added to the fermentation tank. At this time, the xylose concentration was about 180g/L. As theoretically calculated, 500g of glucose was to be added to provide sufficient coenzyme NADPH. Since there was some glucose in the mixed liquid, the xylose secondary mother liquor was needed to replenish remaining glucose by batch feed, totaling 2.7L of xylose secondary mother liquor to be replenished. About 0.8L of corn steep liquor dry powder liquid was batch-fed. The working volume was about 10.5L after the fermentation was completed.

To the fermentation system, a total of 1465g of xylose was added, saving 500g of glucose.

HPLC detection was performed on the fermentation liquid after the fermentation of the control embodiment to obtain a xylitol concentration of 108.2g/L, a conversion rate of 78%, arabinose residues of 5.3g/L and xylose residues of 6.4g/L in the fermentation liquid, and no heterosaccharides such as glucose and mannose were detected.

The test data of the above embodiments and control embodiments are summarized and listed with comparison results shown in Table 3.

**Table 3 Comparison of test results of the xylose fermentation liquids in the embodiments and control embodiments**

| | Xylitol concentration (g/L) | Conversion rate (%) | Arabinose or xylose residues (g/L) | Addition amount of xylose (g) | saving amount of glucose (g) |
|---|---|---|---|---|---|
| Embodiment 1 | 121.4 | 87 | 5.5 (arabinose) | 1465 | 500 |
| Embodiment 2 | 114.1 | 86 | 5.4 (arabinose) | 1657 | 590 |
| Embodiment 3 | 122.4 | 89 | 5.6(arabinose) | 1511 | 530 |
| Embodiment 4 | 125.6 | 87 | 5.8 (arabinose) | 1588 | 550 |
| Control embodiment 1 | 124.9 | 82 | 3.6 (arabinose) | 1600 | / |
| Control embodiment 2 | 58.6 | 41 | 2.2 (arabinose) | 1358 | / |
| Control embodiment 3 | 108.2 | 78 | 5.3 (arabinose), 6.4(xylose) | 1465 | 500 |

It can be known from the control embodiment 1 and the embodiment 1 that it is required to consume a large amount of glucose for xylitol fermentation in the fermentation tank. By batch-feeding the xylose secondary mother liquor, the conversion of xylitol can be satisfied without adding glucose. Further, the xylose secondary mother liquor does not need to be sterilized, resulting in simple operation and easy implementation.

It can be known from the control embodiment 2 and the embodiment 2 that during a xylitol fermentation process, it is necessary to batch-feed glucose because shortage of glucose will reduce the xylitol conversion rate. The effect of batch-feeding the xylose secondary mother liquor is similar to that of adding glucose: on one hand, glucose consumption and costs are reduced; on the other hand, the replenished xylose can increase the xylose concentration in the system, further increasing the conversion rate.

It can be known from the control embodiment 3 and the embodiment 3 that during a xylitol fermentation process, one feed enables the content of the initial xylose or the like to be excessively high, resulting in inhibition on strain growth and affecting conversion rate. Further, xylose residues are detected in the fermentation liquid, and may affect subsequent purification.

The above descriptions are made only to preferred embodiments of the present invention and such embodiments are not intended to limit the present invention. Any modifications, equivalent substitutions and improvements made within the spirit and principle of the present invention shall all be encompassed into the scope of protection of the present invention.

## Claims

1. A method of preparing xylitol by using a xylose secondary mother liquor through fermentation, comprising the following steps:
at step 1, xylose concentrate preparation: adding a base liquid to a pre-ion exchange liquid of a xylose hydrolysate, then performing decolorization using an activated carbon to obtain a fine xylose liquid by filtration, and then performing low-temperature vacuum concentration until a concentrate refractive index is about 60% to 65% so as to obtain a xylose concentrate;
at step 2, strain activation and seed liquid preparation: selecting recombinant escherichia coli strains IS5-M, activating the strains by using an LB plate and an LB liquid medium to inoculate into a seed medium and cultivate to OD₆₀₀=5 to 6 so as to obtain a seed liquid;
at step 3, fermentation tank propagation: adding a sterilized fermentation medium to a fermentation tank and adding the seed liquid to cultivate to OD₆₀₀>20;
at step 4, first batch feed fermentation: adding a mixed liquid obtained by mixing the xylose concentrate and the xylose secondary mother liquor at a ratio to the fermentation tank to perform feed fermentation, such that a xylose concentration in a fermentation system is maintained at 85g/L to 90g/L; when a glucose concentration in the system is less than 10g/L, batch-feeding the xylose secondary mother liquor to control the glucose concentration to 10g/L, where a total glucose addition amount is 35% to 40% of a xylose mass; during the fermentation process, batch-feeding 0.2L to 0.5L of a high concentration solution prepared by using 120g to 130g of a corn steep liquor dry powder liquid at the same time;
at step 5, second batch feed fermentation: when the xylose concentration in the first batch fermentation is <30g/L, performing the second batch feed fermentation using the method in step 4 until a xylitol concentration in the fermentation liquid has no change.

2. The method of claim 1, wherein the step 1 specifically comprises: adding Ca(OH)₂ of 0.4% to 0.5% to the pre-ion exchange liquid of the xylose hydrolysate, and stirring at a speed of 30rpm to 50rpm for 15min to 30min to control pH to 3.0 to 3.5, and then adding the activated carbon of a mass ratio of 0.3% to 0.5% for performing decolorization with a control temperature of 60°C to 70°C and stirring at a speed of 30rpm to 60rpm for 60min to 90min.

3. The method of claim 1, wherein, in step 1, the conditions of the low-temperature vacuum concentration are that a vacuum pump pressure is -0.085MPa to -0.095MPa, a water bath temperature is 60°C to 70°C, a rotation speed is 50rpm to 60rpm, and a condensate flow rate is 150mL/min to 200mL/min.

4. The method of claim 1, wherein in step 2, the strains are recombinant escherichia coli IS5-M.

5. The method of claim 1, wherein in step 2, ingredients of the LB liquid medium are peptone 10g/L, yeast powder 5g/L, sodium chloride 10g/L and solid agar 1.5% to 2%; ingredients of the seed medium are: peptone 7.5g/L, yeast powder 7.5g/L, sodium chloride 10g/L, and glucose 20g/L.

6. The method of claim 1, wherein in step 3, ingredients of the fermentation medium are: glucose 10g/L, corn steep liquor dry powder 24g/L, sodium chloride 0.5g/L, KH₂PO₄ 3g/L, Na₂HPO₄•12H₂O 9g/L and NH₄Cl 1g/L; cultivation conditions of the fermentation tank are: 37°C, 400rpm, pH6.5-7, dissolved oxygen 30% to 35%.

7. The method of claim 1, wherein in step 3, the cultivation lasts for 9.5h to 11h.

8. The method of claim 1, wherein in step 4, a mixing ratio of the xylose concentrate to the xylose secondary mother liquor is 7to 9:1 to 3.

9. The method of claim 1, wherein in step 4, fermentation conditions of the first batch feed fermentation are: 30°C, pH7, dissolved oxygen 20% to 25%.

10. The method of claim 1, wherein the entire fermentation time is about 75h to 85h.
